Europäisches Patentamt

European Patent Office

Office européen des brevets

(19)

(11) Veröffentlichungsnummer : **0 008 151**
**B1**

(12) **EUROPÄISCHE PATENTSCHRIFT**

(45) Veröffentlichungstag der Patentschrift :
**22.09.82**

(21) Anmeldenummer : **79200458.2**

(22) Anmeldetag : **16.08.79**

(51) Int. Cl.³ : **G 01 N 33/00**, G 01 N 25/00,
G 01 K 17/00

(54) **Meßvorrichtung zur Bestimmung einer mit der Wobbezahl eines Gases oder Gasgemisches korrelierten Grösse sowie Verfahren zum Konstanthalten der Wobbezahl von Gasgemischen.**

(30) Priorität : **16.08.78 NL 7808476**

(43) Veröffentlichungstag der Anmeldung :
**20.02.80 (Patentblatt 80/04)**

(45) Bekanntmachung des Hinweises auf die Patenterteilung : **22.09.82 Patentblatt 82/38**

(84) Benannte Vertragsstaaten :
**AT BE DE FR GB IT NL**

(56) Entgegenhaltungen :
**BE A 642 462**
**DE A 2 736 445**
**DE A 2 744 871**

(73) Patentinhaber : **N.V. NEDERLANDSE GASUNIE**
**Postbus 19**
**NL-9700 MA Groningen (NL)**

(72) Erfinder : **Krijgsman, Arie**
**Swarte Ruiters 25**
**NL-9351 NL Leek (NL)**

(74) Vertreter : **Stuffken, Jan et al**
**OCTROOIBUREAU DSM Postbus 9**
**NL-6160 MA Geleen (NL)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

## Meßvorrichtung zur Bestimmung einer mit der Wobbezahl eines Gases oder Gasgemisches korrelierten Größe sowie Verfahren zum Konstanthalten der Wobbezahl von Gasgemischen

Die Erfindung betrifft eine Messvorrichtung zur Bestimmung einer mit der Wobbezahl eines Gases oder Gasgemisches korrelierten Grösse sowie ein Verfahren zum Konstanthalten der Wobbezahl von Gasgemischen unter Verwendung der Messvorrichtung.

Die Wobbezahl W eines Heizgases ergibt sich aus der Beziehung :

$$W = \frac{H_o}{\sqrt{d}}$$

Darin sind :

$H_o$ = der obere Heizwert des Gases in MJ pro m³,

d = die relative Dichte des Gases gegenüber Luft.

Die Wobbezahl ist in der Heiztechnik eine wichtige Grösse, weil bei Speisung eines gasgeheizten Apparates mit unterschiedlich zusammengesetztem Gas unter gleichem Druck die Wärmeerzeugung gleich bleibt und auch der Apparat nicht nachgestellt zu werden braucht, wenn nur die Wobbezahl stets die gleiche ist. Wenn man zum Beispiel in einem Industriebetrieb ein Gemisch von Gasen verschiedener Herkunft verfeuert, muss man, um einen störungsfreien Betrieb zu ermöglichen, die Gase in einem solchen Verhältnis vermischen — evtl. unter Beimischung eines Inertgases (als welches in diesem Zusammenhang auch Luft gelten dürfte), dass ein Gas mit konstanter Wobbezahl erhalten wird.

Zur Bestimmung der Wobbezahl eines Gases benutzt man meistens eine ziemliche kostspielige Kombination von Geräten, bestehend aus einem Kalorimeter, einem Dichtemesser und einer Rechenschaltung bezw. einem Rechenmechanismus. Diese Teile können ggf. zu einem einzigen Messgerät zusammengebaut werden. Ein solches Gerät ist nicht nur kostspielig sondern funktioniert auch träge, so dass schnelle Änderungen in der Gaszusammensetzung nur langsam gemessen und eventuelle Korrekturen nur langsam eingeleitet werden. Aufgabe der vorliegenden Erfindung ist eine preiswertere Messapparatur für insbesondere einen schnelleren Mess- und Regelvorgang der Wobbezahl zu schaffen.

Dieser Mess- bezw. Regelvorgang beruht auf der Feststellung, dass, wenn Gase von unterschiedlicher Zusammensetzung und mit abweichender Wobbezahl mit gleichen Luftmengen verbrannt werden, der Sauerstoffgehalt der Rauchgase eine direkte Korrelation mit der Wobbezahl aufweist. Für Mess- und Regelzwecke muss man somit die Wobbezahl nicht als solche messen, es genügt vielmehr die Messung des Sauerstoffgehalts in diesen Rauchgasen. Die erfindungsgemässe Messvorrichtung kennzeichnet sich durch eine Probenahmeleitung zur Entnahme eines Probegasstromes, in der sich eine Drosselstelle befindet, Mittel zur derartigen Regelung des Probegasstromes, dass der Differenzdruck über dieser Drosselstelle einen eingestellten konstanten Wert hat, Mittel für die Zumischung eines konstanten Volumenstromes Verbrennungsluft zum Probegasstrom, eine Verbrenungskammer, einen Brenner in der Verbrennungskammer zur vollständiger Verbrennung des Gas-Luftgemisches, eine Ableitung für die Abgase aus der Verbrennungskammer, und ein Sauerstoffmessgerät zur Messung des Sauerstoffgehaltes der Abgase. Dieser Sauerstoffgehalt ist eine mit der Wobbezahl des geprüften Gases korrelierte Grösse. Die Korrelation zwischen Wobbezahl und $O_2$-Gehalt wird im Untenstehenden noch näher erörtert. Als wesentlicher Vorteil der Erfindung ist dabei der Einsatz eines einfachen Messgerätes statt eines kostspieligen Wobbezahlmessgerätes zu betrachten.

Die genannten Mittel zur Regelung der Probegasstromes bestehen vorzugsweise aus einem Differenzdruck-Messer/Regler zur Messung der Druckunterschieds über der Drosselstelle sowie einem von diesem Messer/Regler gesteuerten Regelventil in der Probenahmeleitung.

Die genannten Mittel zur Zuführung eines konstanten aus Verbrennungsluft bestehenden Volumenstroms bestehen vorzugsweise aus einer Luftzuleitung mit Drosselstelle, einem Messgerät/Regler zur Messung des Differenzdruckes über dieser Drosselstelle und einem von diesem Messgerät/Regler gesteuerten Regelventil in der Luftzuleitung. Die Luftzuleitung kann dabei an jede geeignete Druckluftquelle, z.B. ein Betriebsluftnetz oder ein Gebläse u.ä. angeschlossen werden. Die Luftmenge kann ausser mit der obigen Kombination von Apparaten auch auf andere an sich bekannte Weise konstant gehalten werden.

Weil die Wobbezahl nur durch zwei Grössen bedingt ist, näml. den Verbrennungswert je m³ und die Heizgasdichte, ist es eine überraschende Erkenntnis, dass durch :

(1) das Regeln des Volumenstroms an Probegas in Abhängigkeit von der Dichte (weil bei konstantem Differenzdruck über eine Drosselstelle der Volumenstrom nur durch die Gasdichte bedingt ist),

(2) die Verbrennung desselben unter konstanter Luftzufuhr, und

(3) das Messen des verbleibenden Sauerstoffgehalts in den Verbrennungsgasen, eine weitaus einfachere Messanlage als die bisher übliche genügt.

Wo in dieser Anmeldung von Drosselstelle die Rede ist, kann es sich um jede in der Praxis übliche Drosselstelle handeln, wie Messblende, Venturirohr usw.

Die Ablesung des Sauerstoffmessgerätes (z.b. Ziffernblatt oder Digitalzifferntafel), ist vorzugsweise so einzurichten, dass sowohl der $O_2$-Gehalt der Verbrennungsgase als auch die darauf bezogene Wobbezahl direkt ablesbar sind.

Bei dem ferner beschriebenen Verfahren zum Mischen von unterschiedlich zusammengesetzten Teilgasströmen zu einem Mischheizgasstrom mit im wesentlichen konstanter Wobbezahl unter Verwendung einer erfindungsgemässen Messvorrichtung, bei dem man dem Mischheizgas eine Probe entnimmt, deren Wobbezahl prüft sowie auf Grund der Prüfergebnisse mindestens einen der Teilströme regelt, wird erfindungsgemäss der zu prüfende Probegasstrom mit Hilfe einer Probenahmeleitung genommen in der sich eine Drosselstelle befindet, wobei der Probegasstrom in der Weise geregelt wird, dass der Differenzdruck über der Drosselstelle einen voraus eingestellten konstanten Wert hat, wird ein konstanter, einen Luftüberschuss bewirkender Volumenstrom an Verbrennungsluft dem Probegas zugemischt, wird das so erhaltene Gas-Luftgemisch in einer Verbrennungskammer vollständig verbrannt, wird der Sauerstoffgehalt der Abgase bestimmt und wird wenigstens einer der Teilgasströme in der Weise geregelt, dass der Sauerstoffgehalt der Abgase einen konstanten gewählten Wert behält.

Die Erfindung wird anhand einer Zeichnung und eines Beispiels erläutert. Es ist

Figur 1 : ein Schema einer erfindungsgemässen Messanlage ;

Figur 2 : ein Diagramm, in dem die Korrelation zwischen dem gemessenen Sauerstoffgehalt und der Wobbezahl des geprüften Gases dargestellt ist ;

Figur 3 : ein Schema einer Anlage zur Zusammensetzung verschiedener Teilströme zu einem Gas mit konstanter Wobbezahl mit Hilfe des erfindungsgemässen Verfahrens.

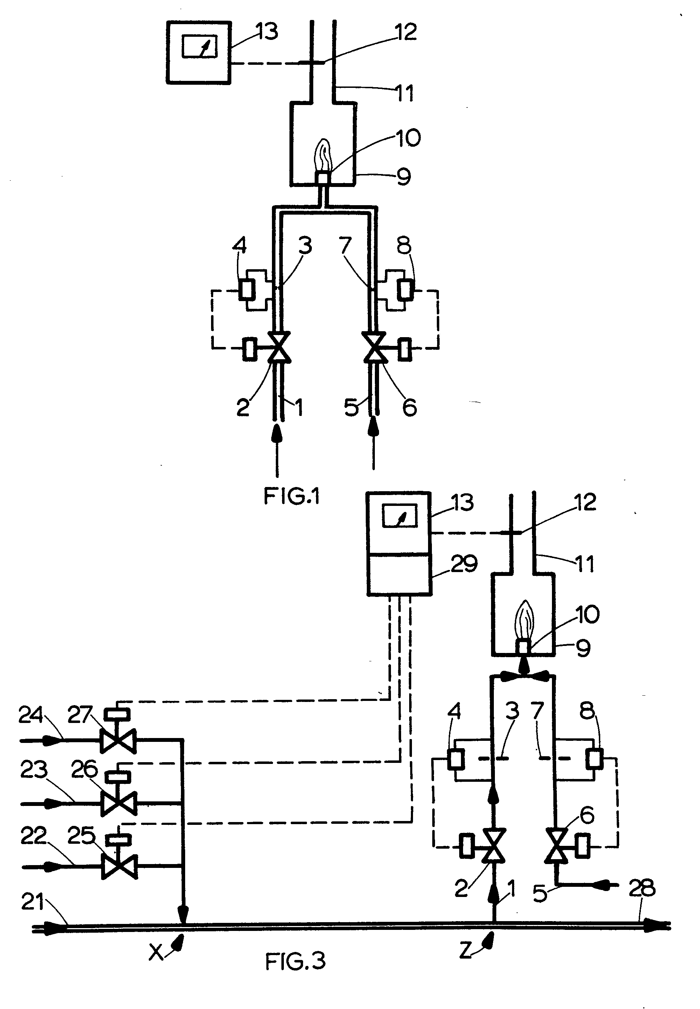

In Figur 1 haben die Bezugsziffern die folgende Bedeutung :

1 : eine Probenahmeleitung für die kontinuierliche Zuführung des Probegases ;

2 : ein Regelventil in Probenahmeleitung 1 ;

3 : eine Drosselstelle in Probenahmeleitung 1 ;

4 : ein Mess-Regelgerät, das den Differenzdruck über der Drosselstelle 3 misst und das Regelventil 2 derart steuert, dass der Differenzdruck einen voraus eingestellten konstanten Wert aufweist ;

5 : eine Luftzuleitung für Verbrennungsluft ;

6 : ein Regelventil in Luftzuleitung 5 ;

7 : eine Drosselstelle in Luftzuleitung 5 ;

8 : ein Mess-Regelgerät, das den Differenzdruck über der Drosselstelle 7 misst und das Regelventil 6 derart steuert, dass der Differenzdruck einen eingestellten konstanten Wert hat, wodurch auch der Volumenstrom an Verbrennungsluft konstant ist ;

9 : eine Verbrennungskammer ;

10 : ein Brenner, in dem das von der Probenahmeleitung 1 herangeführte Probegas mit der Verbrennungsluft aus der Luftzuleitung 5 vollständig verbrannt wird ;

11 : eine Leitung zur Abführung der Abgase ;

12 : ein Messelement zum Messen des Sauerstoffgehalts der Abgase ;

13 : ein an das Messelement 12 angeschlossenes Sauerstoffmessgerät, ausgestattet mit Ziffernblatt, auf dem nicht nur der gemessene Sauerstoffgehalt sondern auch die ihm zugeordnete Wobbezahl angezeigt werden.

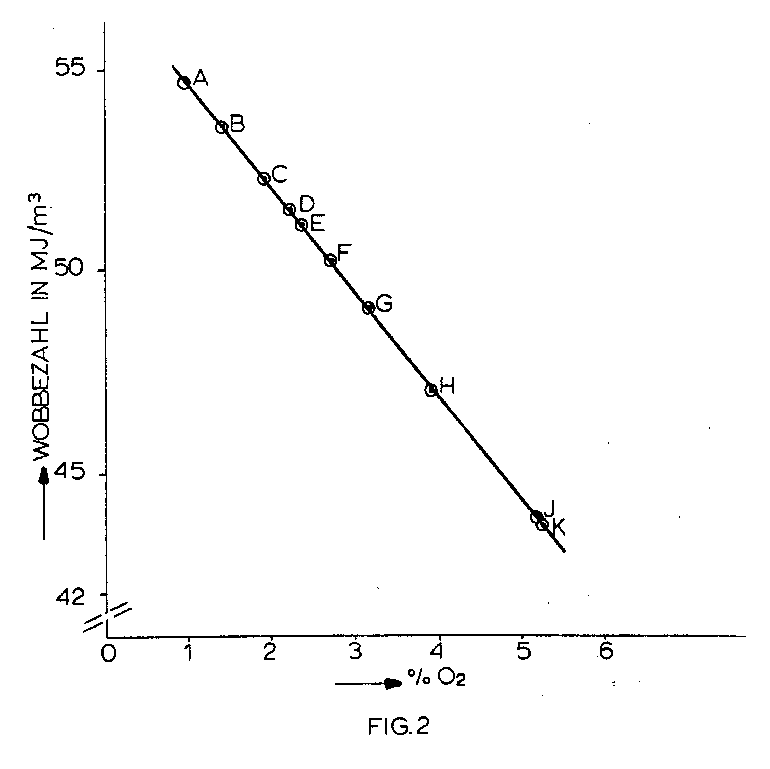

In Diagramm Fig. 2 ist die Korrelation zwischen der Wobbezahl und dem ermittelten Sauerstoffgehalt dargestellt. Für 10 Gase A-K, im wesentlichen bestehend aus Kohlenwasserstoffen mit einem grösseren oder geringeren Inertgasanteil, wie $N_2$ and $CO_2$, wurde auf konventionelle Weise die Wobbezahl und ausserdem der Sauerstoffgehalt in den Abgasen bei Anwendung der erfindungsgemässen Messanlage ermittelt. Diese Werte wurden graphisch gegeneinander abgetragen, näml. der Sauerstoffgehalt auf der Waagerechten und die Wobbezahl auf der Senkrechten.

Die Punkte A, C, D, E, F, J und K betreffen Erdgase aus verschiedenen Feldern :

B ist reines Methan,

G und H sind Gemische von J mit Propan,

Punkt J ist Gas aus dem Slochteren-Vorkommen in Groningen,

Die Luftzufuhr durch die Luftleitung 5 (Fig. 1) ist so eingestellt, dass für J der Luftfaktor 1,3 beträgt, d.h. : die Zuluft ist das 1,3 fache der für eine stöchiometrisch vollständige Verbrennung benötigten Menge.

Wie man sieht, ist die Korrelation zwischen Sauerstoffgehalt und Wobbezahl für hauptsächlich aus Kohlenwassenstoffen und Inertgasen bestehende Gase innerhalb des geprüften Abschnittes linear und ist die Korrelation zwischen $O_2$-Gehalt und Wobbezahl sehr gut.

In Fig. 3 haben die Bezugsziffern 1 bis 13 die gleiche Bedeutung wie in Fig. 1. Die übrigen Bezugsziffern haben die folgende Bedeutung :

21, 22, 23, 24 : Zuleitungen für verschiedene zu mischende Gasarten ;

25, 26, 27 : Regelventile in den Leitungen 22-24 ;

28 : eine Ableitung für vermischtes Heizgas ;

29 : ein Regler, der vom Sauerstoffmessgerät 13 ein Signal empfängt und die Regelventile 22-24 so steuern kann, dass der durch das Sauerstoffmessgerät 13 gemessene Sauerstoffgehalt auf dem eingestellten Wert konstant gehalten wird.

Der durch die Leitung 21 zuströmende Teilstrom hat in diesem Falle den Charakter eines Hauptstromes, dem durch eine oder mehrere der Leitungen 22-24 eine oder mehrere Teilströme zur Berichtigung der Wobbezahl zugesetzt werden können. Der Punkt X, wo die Einspeisung, und der Punkt Z, wo die Probenahme stattfindet, müssen so weit auseinander liegen, dass die Gase in Punkt Z im wesentlichen völlig vermischt sind. In Fig. 3 sind vier Teilstromzuleitungen 21-24 dargestellt.

Häufig wird neben einer Hauptstromleitung 21 nur eine sekundäre Teilstromleitung (z.B. 22) vorhanden sein. Wenn dann der Hauptstrom aus einem Gas mit einer stets über dem Sollwert liegenden Wobbezahl besteht, wird als Sekundarteilstrom ein Gas mit niedriger Wobbezahl oder ein Inertgas zugesetzt ; wenn der Hauptstrom aus einem Gas mit einer stets unter dem Sollwert liegenden Wobbezahl besteht, wird als Sekundarteilstrom ein Gas mit höherer Wobbezahl zugesetzt. Wenn die Wobbezahl des Hauptstromes den Sollwert sowohl über- als unterschreiten kann, können zwei Sekundarteilstromzuleitungen, z.B. 22 und 23, vorhanden sein, wobei dann durch die eine Sekundarzuleitung Gas mit einer hohen Wobbezahl und durch die andere Gas mit einer niedrigen Wobbezahl mit Hilfe der Steuerung durch den Regler 29 nach Bedarf zugeführt werden können.

Beispiel

Das erfindungsgemässe Verfahren wurde angewandt zur Erhaltung von Heizgasgemischen mit konstanter Wobbezahl aus dem Groninger Erdgas und/oder dem Erdgas Middelie 2, das eine niedrige Wobbezahl hat, und/oder dem Ekofiskfeld, das eine hohe Wobbezahl hat, und/oder Propan, das eine sehr hohe Wobbezahl hat, evtl. unter Beimischung von Inertgas, als welches im vorliegenden Fall Luft verwendet wurde. Die Ergebnisse sind in untenstehender Tabelle erfasst, in der die Volumenanteile, in denen die zugeführten Gase vermischt wurden, in Prozent sowie auch die mit einem üblichen Wobbezahlmessgerät gemessenen Wobbezahlen zur Überprüfung der Mischungen aufgeführt sind.

Die Regelung der Volumen der zu vermischenden Gase war stets derart, dass der Sauerstoffgehalt des aus dem verbrannten Probegas herrührenden Abgases immer ca. 5,2 Vol.-% betrug ; dies ist der bei Verbrennung mit Luftfaktor 1,30 für Groninger Erdgas geltende Wert. Wie aus der Tabelle ersichtlich ist, erhält man ein Mischgas mit sehr befriedigender konstanter Wobbezahl.

Gasgemische zusammengesetzt aus :

| Gasgemisch Nr. | Groninger Erdgas Vol.-% | Ekofisk Vol.-% | Midellie 2 Vol.-% | Propan Vol.-% | Inertgas (= Luft) Vol.-% | Wobbezahl des Gemisches MJ/m$^3$ |
|---|---|---|---|---|---|---|
| I | 87,52 | 10,45 | — | — | 2,03 | 43,90 |
| II | 47,56 | 43,91 | — | — | 8,53 | 43,95 |
| III | 29,41 | 59,09 | — | — | 11,50 | 43,95 |
| IV | — | 57,75 | 42,25 | — | — | 43,85 |
| V | 74,33 | — | — | 13,1 | 12,57 | 43,95 |
| Wobbezahl | 43,85 | 54,60 | 31,37 | 81,06 | — | — |

**Ansprüche**

1. Messvorrichtung zur Bestimmung einer mit der Wobbezahl eines Gases korrelierten Grösse, gekennzeichnet durch eine Probenahme tung (1) zur Entnahme eines Probegasstromes, in welcher Leitung sich eine Drosselstelle (3) befindet, Mittel (2, 4) zur derartigen Regelung des Probegasstromes, dass der Differenzdruck über der Drosselstelle einen eingestellten konstanten Wert hat, Mittel (5, 6, 7, 8) für die Zumischung eines konstanten Volumenstromes Verbrennungsluft zum Probegasstrom, eine Verbrennungskammer (9), einen Brenner (10) in der Verbrennungskammer zur vollständigen Verbrennung des Gas-Luftgemisches, eine Ableitung (11) für die Abgase aus der Verbrennungskammer und ein Saurstoffmessgerät (12, 13) zum Messen des Sauerstoffgehalts der Abgase.

2. Messvorrichtung gemäss Anspruch 1, dadurch gekennzeichnet, dass die genannten Mittel zur Regelung des Probegasstromes aus einem Differenzdruck-Messer/Regler (4) zum Messen des Differenzdruckes über der Drosselstelle (3) sowie einem von diesem Messer/Regler gesteuerten Regelventil (2) in der Probenahmeleitung (1) bestehen.

3. Messvorrichtung gemäss Anspruch 1 oder 2, dadurch gekennzeichnet, dass die genannten Mittel für Zuführung eines konstanten Volumenstromes Verbrennungsluft aus einer Luftzuleitung (5), in der sich eine Drosselstelle (7) befindet, einem Differenzdruck-Messer/Regler (8) zum Messen des Differenzdruckes über dieser Drosselstelle und einem von diesem Messer/Regler (8) gesteuerten Regelventil (6) in der Luftzuleitung (5) bestehen.

4. Verfahren zum Mischen von unterschiedlich zusammengesetzten Teilgasströmen zu einem Mischheizgasstrom mit einer im wesentlichen konstanten Wobbezahl unter Verwendung einer Messvorrichtung gemäss einem der Ansprüchen 1, 2 oder 3, bei dem man dem Mischheizgasstrom einen Probengasstrom entnimmt, dessen Wobbezahl prüft und in Abhängigkeit vom Ergebnis dieser Prüfung zumindest einen der Teilgasströme regelt, dadurch gekennzeichnet, dass der zu prüfende Probegasstrom mit Hilfe einer Probenahmleitung (1) genommen wird, in der sich eine Drosselstelle (2) befindet, wobei der Probegasstrom in der Weise geregelt wird, dass der Differenzdruck über der Drosselstelle (2) einen im voraus eingestellten Wert aufweist, dass ein konstanter, einen Luftüberschuss bewirkender Volumenstrom an Verbrennungsluft dem Progegasstrom zugemischt, das so erhaltene Gas-Luft-Gemisch in einer Verbrennungskammer (9) vollständig verbrannt, der Sauerstoffgehalt der Abgase bestimmt und zumindest einer der Teilgasströme in der Weise geregelt wird, dass der Sauerstoffgehalt der Abgase einen konstanten gewählten Wert behält.

## Claims

1. Measuring system for determining a quantity correlated with the Wobbe index of a gas, this system being characterized by a sampling line (1) for the supply of a flow of the gas to be tested, which line incorporates a restriction (3), means (2, 4) for so controlling the flow of this gas that the pressure differential across the restriction can be given a constant, pre-set value, means (5, 6, 7, 8) for adding a constant-volume flow of combustion air to the flow of gas to be tested, a combustion chamber (9), a burner (10) in the combustion chamber for complete combustion of the gas/air mixture, a discharge line (11) for combustion gases leaving the combustion chamber, and an oxygen meter (12, 13) for measuring the oxygen content of the combustion gases.

2. Measuring system according to claim 1, characterized in that the said means for controlling the flow of gas to be tested consist of a pressure differential meter/controller (4) for measuring the pressure differential across the restriction (3), and a control valve (2) in the sampling line (1), controlled by the meter/controller.

3. Measuring system according to claim 1 or 2, characterized in that the said means for supply of a constant-volume flow of combustion air consist of an air supply line (5) incorporating a restriction (7), a pressure differential meter/controller (8) for measuring the pressure differential across. this restriction, and a control valve (6) in the air supply line (5), controlled by this meter/controller.

4. Process of mixing part-flows of gases differing in composition to form a mixed fuel-gas flow having a substantially constant Wobbe index, using a measuring system according to claim 1, 2 or 3, by taking a sample of the mixed fuel gas so formed and testing this sample, with at least one of the part-flows being controlled with reference to this test, this process being, characterized in that the flow of gas to be tested is supplied along a sampling line (1) incorporating a restriction (2), the flow of gas to be tested being so controlled that the pressure differential across the restriction (2) has a pre-set value, an excess, constant-volume flow of combustion air is added to the gas to be tested, the resulting gas/air mixture is completely combusted in a combustion chamber (9), the oxygen content of the combustion gases is determined, and at least one of the part-flows is so controlled that the oxygen content of the combustion gases retains a constant, pre-selected value.

## Revendications

1. Dispositif de mesure pour déterminer une grandeur étant en corrélation avec l'indice de Wobbe d'un gaz, caractérisé par une conduite (1) servant à prélever un courant de gaz-échantillon, laquelle conduite est munie d'un étranglement (3), par des moyens (2, 4) servant à régler le courant de gaz-échantillon de telle façon que la différence de pression sur l'étranglement puisse avoir une valeur constante réglée, par des moyens (5, 6, 7, 8) servant à amener un volume constant d'air de combustion au courant de gaz-échantillon, par une chambre de combustion (9), par un brûleur (10) dans la chambre de combustion pour assurer la combustion complète du mélange gaz-air, par une conduite d'évacuation (11) pour évacuer les gaz de combustion de la chambre de combustion et par un compteur d'oxygène (12, 13) pour mesurer la teneur en oxygène des gaz de combustion.

2. Dispositif de mesure selon la revendication 1, caractérisé par le fait que lesdits moyens servant à régler le courant de gaz-échantillon, se composent d'un mesureur/régulateur de la différence de pression (4) pour mesurer la différence de pression sur l'étranglement (3) et d'une soupape de réglage (2), commandée par ce mesureur/régulateur montée dans la conduite (1) servant à prélever le courant de gaz-échantillon.

3. Dispositif de mesure selon l'une des revendications 1 à 2, caractérisé en ce que lesdits moyens servant à amener un volume constant d'air de combustion se composent d'une conduite d'amenée d'air (5) munie d'un étranglement (7), d'un mesureur-régumateur (8) pour mesurer la différence de pression sur cet étranglement et d'une soupape de réglage (6) montée dans la conduite d'amenée d'air (5) et commandée par ce mesureur-régulateur (8).

4. Procédé pour mélanger des courants partiels de compositions différente en un courant de gaz de chauffage composé avec un indice de Wobbe essentiellement constant, pour lequel on utilise un dispositif de mesure selon l'une des revendications 1, 2 et 3, en prélevant et en examinant un échantillon à partir du gaz de chauffage composé et en réglant du moins un de ces courants partiels à l'aide des résultats de cet examen, caractérisé en ce que le courant du gaz-échantillon à examiner est prélevé à l'aide d'une conduite de prélèvement d'échantillons (1) muni d'un étranglement (2), tout en réglant le courant-échantillon de telle façon que la différence de pression sur cet étranglement (2) ait une valeur constante réglée à l'avance, qu'un volume constant d'air de combustion soit amené en excès au gaz-échantillon, que le mélange obtenu de gaz-air soit complètement brûlé dans une chambre de combustion (9), que la teneur en oxygène des gaz de combustion soit déterminée et que du moins un des courants partiels soit réglé de telle façon que la teneur en oxygène des gaz de combustion ait une valeur constante choisie.

FIG.1

FIG.3

FIG.2